# EUROPEAN PATENT SPECIFICATION

(11) **EP 3 231 351 B1**
(45) Date of publication and mention of the grant of the patent: **30.06.2021**
(21) Application number: 15868333.4
(22) Date of filing: 05.11.2015
(51) Int. Cl.: A61B 1/04, G02B 23/24, H04N 5/225, A61B 1/00, A61B 1/05

(54) **IMAGING UNIT, ENDOSCOPE, AND METHOD FOR PRODUCING IMAGING UNIT**
BILDGEBUNGSEINHEIT, ENDOSKOP UND VERFAHREN ZUR HERSTELLUNG DER BILDGEBUNGSEINHEIT
UNITÉ D'IMAGERIE, ENDOSCOPE ET PROCÉDÉ DE PRODUCTION D'UNITÉ D'IMAGERIE

(30) Priority: 08.12.2014 JP 2014248176
(43) Date of publication of application: 18.10.2017
(73) Proprietor: Olympus Corporation, Hachioji-shi, Tokyo 192-8507 (JP); Nuvoton Technology Corporation Japan, Nagaokakyo-shi, Kyoto 617-8520 (JP)
(72) Inventor: YAMASHITA, Tomokazu, Tokyo 192-8507 (JP); IGARASHI, Takatoshi, Tokyo 192-8507 (JP); FUJIMORI, Noriyuki, Tokyo 192-8507 (JP); TAKAYAMA, Yoshiki, Nagaokakyo-shi Kyoto 617-8520 (JP); HARADA, Yutaka, Nagaokakyo-shi Kyoto 617-8520 (JP)
(74) Representative: Gunzelmann, Rainer
(86) International application number: PCT/JP2015/081195
(87) International publication number: WO 2016/092992

(56) References cited:
- EP-A1- 2 433 996
- GB-A- 2 321 132
- JP-A- 2010 268 077
- JP-A- 2011 050 496
- JP-A- 2012 183 330
- US-B1- 6 447 445
- None

## Description

### Field

The present invention relates to an imaging unit, an endoscope including an imaging unit, and a method of manufacturing an imaging unit.

### Background

Conventionally, endoscopes have been widely used for various examinations in the medical field and the industrial field. Among them, endoscopes for medical use have been widely used since an in-vivo image of a body cavity of a subject such as a patient can be acquired without the need for making an incision in the subject by inserting, into the body cavity of the subject, an elongated flexible insertion portion including a distal end provided with a solid state image sensor, and medical treatment can be performed by causing a treatment tool to project from the distal end of the insertion portion as necessary.

With regard to an imaging device that is used in such an endoscope, a technique for reducing the size of the imaging device by making the size of each of a plurality of electronic components mounted on a multi-layer substrate equal to or less than the length in a lateral direction (width direction) of a flexible substrate or the multi-layer substrate has been disclosed (for example, refer to Patent Literature 1). In this example, a bypass capacitor for an image sensor is mounted on a first circuit board that is the flexible substrate, and a recessed portion for housing the bypass capacitor for the image sensor is provided on the opposite multi-layer substrate.

JP2010268077 disclosing an imaging device which comprises a solid-state image sensor and several electronic components mounted in a flexible substrate.

### Patent Literature

Patent Literature 1: JP 2011-50496 A

### Summary

### Technical Problem

In the technique described in Patent Literature 1, however, since the height of the recessed portion needs to be deeper than the height of the component, the thickness of the multi-layer substrate is reduced at the part provided with the recessed portion, and the multi-layer substrate is likely to break.

The present invention has been made in consideration of the above-mentioned circumstances, and an object thereof is to provide an imaging unit, an endoscope, and a method of manufacturing an imaging unit for preventing breakage of a multi-layer substrate while achieving a reduction in size. Solution to Problem

To solve the problem described above and to achieve the object, an imaging unit according to the present invention includes the features of claim 1.

In the imaging unit according to the present invention, two or more of the electronic components having different heights are mounted in the recessed portion, and the tallest electronic component is configured not to project from the surface of the flexible substrate opposite to the connection surface of the flexible substrate connected to the multi-layer substrate.

In the imaging unit according to the present invention, the two electronic components having different heights are mounted in the recessed portion, and the two electronic components are mounted side by side in a longitudinal direction of the multi-layer substrate.

In the imaging unit according to the present invention, cable connection lands are formed on proximal end sides relative to the recessed portion on the connection surface of the multi-layer substrate and a surface of the multi-layer substrate opposite to the connection surface. A drive signal cable and an image signal cable are connected to the cable connection lands formed on the different surfaces of the multi-layer substrate, respectively.

An endoscope according to the present invention includes the features of claim 4.

A method of manufacturing an imaging unit according to the present invention is a method of manufacturing any one of the above-described imaging units. The method includes the steps of claim 5.

The method of manufacturing the imaging unit according to the present invention further includes a sealing step of performing between the mounting step and the cutting step, the sealing step including filling and sealing, with a sealing resin, the recessed portion in which the electronic component has been mounted.

### Advantageous Effects of Invention

The present invention can provide an imaging unit, an endoscope, and a method of manufacturing an imaging unit for realizing a reduction in diameter while suppressing breakage of a multi-layer substrate.

### Brief Description of Drawings

FIG. 1 is a view schematically illustrating an overall configuration of an endoscope device according to an embodiment of the present invention.
FIG. 2 is a partial cross-sectional view of a distal end of an endoscope illustrated in FIG. 1.
FIG. 3 is a top view of an imaging unit illustrated in FIG. 2.
FIG. 4 is another view of FIG. 3 seen in the direction of arrow A.
FIG. 5 is another view of FIG. 3 seen in the direction of arrow B.
FIG. 6 is another view of FIG. 3 seen in the direction of arrow C.
FIG. 7 is a bottom view of the imaging unit illustrated in FIG. 2 (another view of FIG. 4 seen in the direction of arrow D).
FIG. 8 is a flowchart describing steps of manufacturing the imaging unit according to the embodiment of the present invention.
FIG. 9 is a left side view of an imaging unit according to a first variation of the embodiment of the present invention.
FIG. 10 is another view of FIG. 9 seen in the direction of arrow F.
FIG. 11 is a left side view of an imaging unit according to a second variation of the embodiment of the present invention.
FIG. 12 is a left side view of an imaging unit according to a third variation of the embodiment of the present invention.
FIG. 13 is another view of FIG. 12 seen in the direction of arrow G.
FIG. 14 is a partial top view of an imaging unit according to a fourth variation of the embodiment of the present invention.
FIG. 15 is another view of FIG. 14 seen in the direction of arrow E.

### Description of Embodiments

In the following description, as an embodiment for practicing the present invention (hereinafter referred to as the "embodiment"), an endoscope equipped with an imaging unit will be described. The present invention is not limited by the embodiment. In the drawings, identical elements are provided with the same reference signs. It should be noted that the drawings are only schematic, and a relation between thickness and width of each member and a ratio of each member or the like are different from actual ones. Dimensions and ratios in the different drawings may also be different from one another.

### (First Embodiment)

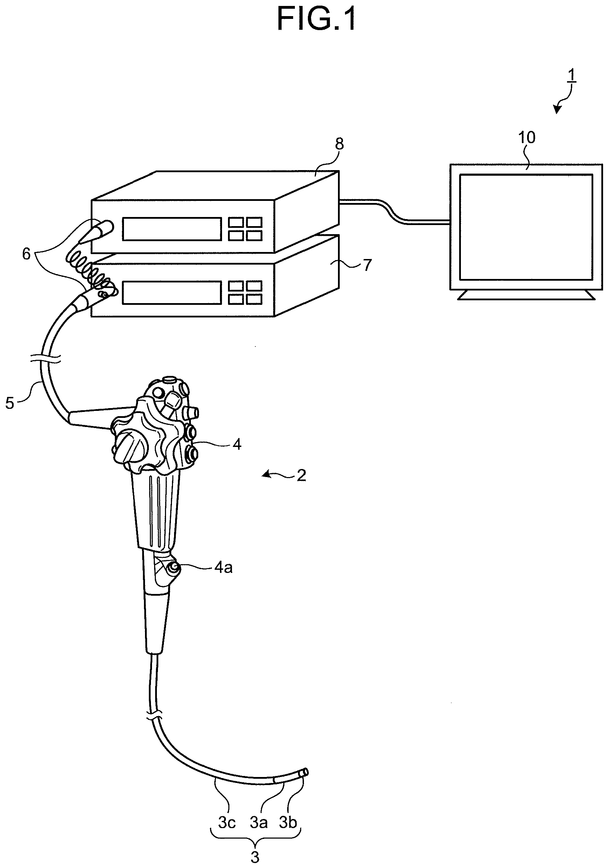

FIG. 1 is a view schematically illustrating an overall configuration of an endoscope device according to a first embodiment of the present invention. As illustrated in FIG. 1, an endoscope device 1 includes an endoscope 2, a universal code 5, a connector 6, a light source device 7, a processor (control device) 8, and a display device 10.

The endoscope 2 captures an in-vivo image of a subject and outputs an imaging signal by inserting an insertion portion 3 into a body cavity of the subject. A cable within the universal code 5 is extended to a distal end of the insertion portion 3 of the endoscope 2 and connected to an imaging unit provided at a distal end portion 3b of the insertion portion 3.

The connector 6 is provided at a proximal end of the universal code 5 and connected to the light source device 7 and the processor 8. The connector 6 performs a predetermined signal process for the imaging signal (output signal) output by the imaging unit at the distal end portion 3b connected to the universal code 5, and subjects the imaging signal to an analog-digital conversion (A/D conversion) to output it as an image signal.

The light source device 7 is configured by using, for example, a white LED. Pulse-like white light shed by the light source device 7 passes through the connector 6 and the universal code 5 to become illumination light radiated from the distal end of the insertion portion 3 of the endoscope 2 toward an object.

The processor 8 performs a predetermined image process for the image signal output from the connector 6, and controls the entire endoscope device 1. The display device 10 displays the image signal processed by the processor 8.

An operating unit 4 is connected to a proximal end side of the insertion portion 3 of the endoscope 2. Various buttons and knobs or the like for operating an endoscope function are provided on the operating unit 4. A treatment tool insertion opening 4a is provided in the operating unit 4. A treatment tool such as living body forceps, an electric scalpel, and an examination probe is inserted into the body cavity of the subject through the treatment tool insertion opening 4a.

The insertion portion 3 includes the distal end portion 3b, a curve portion 3a, and a flexible pipe portion 3c. The imaging unit is provided at the distal end portion 3b. The curve portion 3a is continuously provided on a proximal end side of the distal end portion 3b and freely curved in an up-down direction. The flexible pipe portion 3c is continuously provided on a proximal end side of the curve portion 3a. The curve portion 3a is curved in the up-down direction by an operation for a curving operation knob provided on the operating unit 4, and freely curved, for example, in two directions including upward and downward directions as a curve wire inserted into the insertion portion 3 is pulled or loosened.

A light guide that delivers the illumination light from the light source device 7 is arranged in the endoscope 2. An illumination window is arranged at an emission end of the illumination light delivered through the light guide. The illumination window is provided at the distal end portion 3b of the insertion portion 3, and the illumination light is radiated toward the subject.

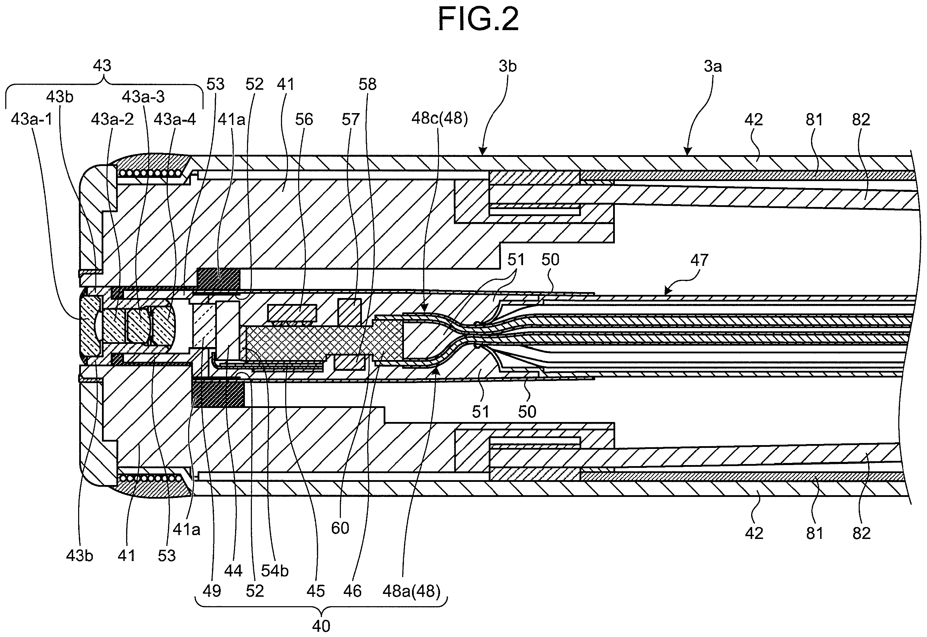

Next, a configuration of the distal end portion 3b of the endoscope 2 will be described in detail. FIG. 2 is a partial cross-sectional view of a distal end of the endoscope 2. FIG. 2 is the cross-sectional view cut off by a plane orthogonal to a substrate surface of the imaging unit provided at the distal end portion 3b of the endoscope 2, and in parallel with an optical axis direction of the imaging unit. In FIG. 2, the distal end portion 3b of the insertion portion 3 of the endoscope 2 and a part of the curve portion 3a are illustrated.

As illustrated in FIG. 2, the curve portion 3a is freely curved in four directions including upward, downward, left, and right directions as a curve wire 82 inserted into a curve pipe 81 arranged inside a covering pipe 42 (to be described later) is pulled or loosened. An imaging unit 40 is provided within the distal end portion 3b provided to extend from a distal end side of the curve portion 3a.

The imaging unit 40 has a lens unit 43 and a solid state image sensor 44 arranged on a proximal end side of the lens unit 43. The imaging unit 40 is bonded to the inside of a distal end portion body 41 by an adhesive. The distal end portion body 41 is formed of a rigid member for forming an internal space in which the imaging unit 40 is housed. An outer peripheral portion of a proximal end of the distal end portion body 41 is covered with the pliable covering pipe 42. Since a member located on a proximal end side relative to the distal end portion body 41 is configured by a pliable member so that the curve portion 3a can be curved, a rigid part of the insertion portion 3 is the distal end portion 3b where the distal end portion body 41 is arranged.

The lens unit 43 has a plurality of objective lenses 43a-1 to 43a-4 and a lens holder 43b that holds the objective lenses 43a-1 to 43a-4. The lens unit 43 is fixed to the distal end portion body 41 when a distal end of the lens holder 43b is inserted to be fit and fixed into the distal end portion body 41.

The imaging unit 40 includes the solid state image sensor 44, a flexible substrate 45, a multi-layer substrate 46, a glass rid 49, and a plurality of signal cables 48. The solid state image sensor 44 such as a CCD and a CMOS includes, on a surface of the solid state image sensor 44, a light receiving surface that receives light. The flexible substrate 45 extends from the solid state image sensor 44. The multi-layer substrate 46 includes electronic components including a drive circuit for the solid state image sensor 44. The electronic components are mounted on the multi-layer substrate 46. The glass rid 49 is bonded to the solid state image sensor 44, with the light receiving surface of the solid state image sensor 44 covered therewith. The plurality of signal cables 48 is electrically connected to the solid state image sensor 44 via the multi-layer substrate 46 for driving the solid state image sensor 44. The multi-layer substrate 46 includes connection lands on which the electronic components are mounted and cable connection lands connected to the signal cables 48. A distal end of the respective signal cables 48 is electrically and mechanically connected to the respective cable connection lands.

The plurality of signal cables 48 is brought together into an electric cable bundle 47 and extends in a proximal end direction of the insertion portion 3. The electric cable bundle 47 is arranged to be inserted into the insertion portion 3, and provided to extend to the connector 6 through the operating unit 4 and the universal code 5 illustrated in FIG. 1.

An object image formed by the objective lenses 43a-1 to 43a-4 of the lens unit 43 is photoelectrically converted by the solid state image sensor 44 arranged at an image forming position of the objective lenses 43a-1 to 43a-4 into the imaging signal that is an electric signal. The imaging signal passes through the signal cables 48 connected to the flexible substrate 45 and the multi-layer substrate 46 and through the connector 6, and is output to the processor 8.

The solid state image sensor 44 is bonded to the flexible substrate 45 and the multi-layer substrate 46 by an adhesive 54b. An outer peripheral portion of the solid state image sensor 44 and a connection portion between the solid state image sensor 44, and the flexible substrate 45 and the multi-layer substrate 46 are covered with a reinforcing member 52 formed of a sleeve-like metal material, both ends of which are open. In order to prevent an influence of static electricity or disturbance noise that flows in from the outside on electronic components 55 to 59 on the multi-layer substrate 46 or a wiring pattern on the flexible substrate 45, the reinforcing member 52 is installed apart from the solid state image sensor 44 and the flexible substrate 45.

Outer peripheries of the imaging unit 40 and a distal end portion of the electric cable bundle 47 are covered with a heat shrinkable tube 50 for improving resistance. The inside of the heat shrinkable tube 50 is configured such that a gap between the components is filled with an adhesive resin 51. An outer peripheral surface of the reinforcing member 52 and an inner peripheral surface of a distal end side of the heat shrinkable tube 50 are in contact with each other without any gap.

A solid state image sensor holder 53 holds the solid state image sensor 44 bonded to the glass rid 49 in such a manner that an outer peripheral surface of the glass rid 49 is fit into an inner peripheral surface of a proximal end side of the solid state image sensor holder 53. An outer peripheral surface of the proximal end side of the solid state image sensor holder 53 is fit with an inner peripheral surface of a distal end side of the reinforcing member 52. An outer peripheral surface of a proximal end side of the lens holder 43b is fit with an inner peripheral surface of a distal end side of the solid state image sensor holder 53. While the respective components are fit with one another in this manner, the outer peripheral surface of the lens holder 43b, the outer peripheral surface of the solid state image sensor holder 53, and an outer peripheral surface of the distal end side of the heat shrinkable tube 50 are fixed to an inner peripheral surface of a distal end of the distal end portion body 41 by an adhesive 41a.

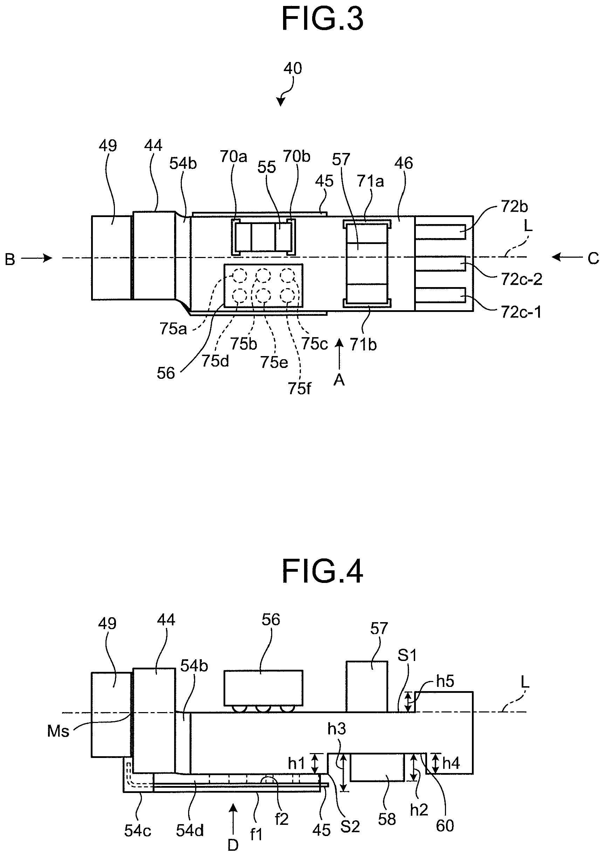

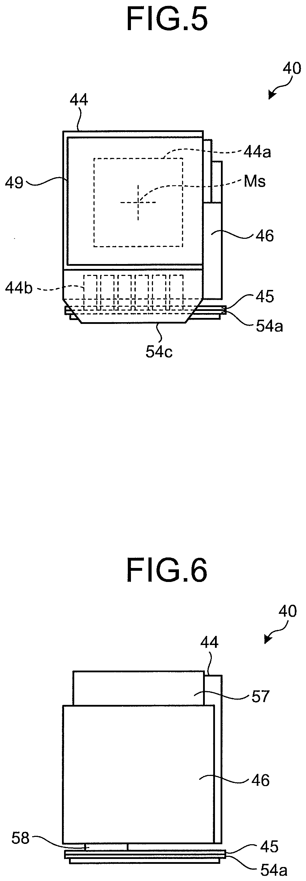

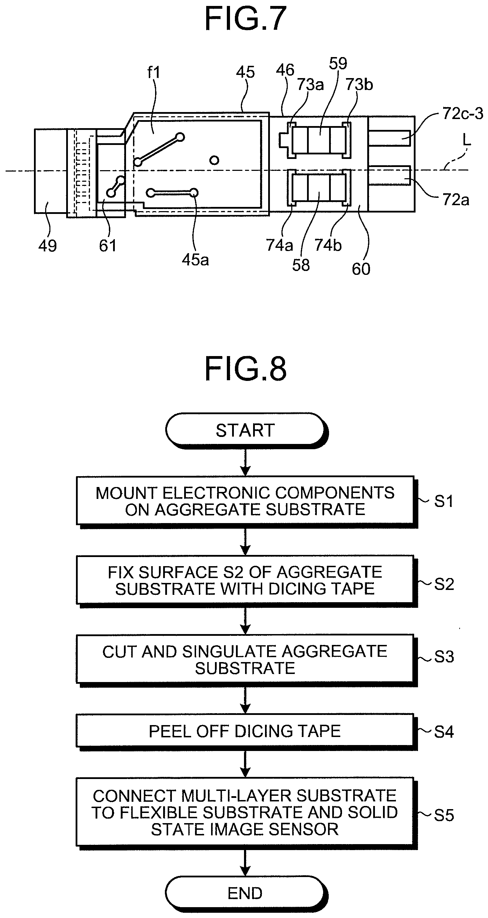

Next, the imaging unit 40 will be described. FIG. 3 is a top view of the imaging unit illustrated in FIG. 2. FIG. 4 is another view of FIG. 3 seen in the direction of arrow A. FIG. 5 is another view of FIG. 3 seen in the direction of arrow B. FIG. 6 is another view of FIG. 3 seen in the direction of arrow C. FIG. 7 is a bottom view of the imaging unit illustrated in FIG. 2 (another view of FIG. 4 seen in the direction of arrow D). In the above drawings, the lens unit 43 and the signal cables 48 are not illustrated, and line L passing through the center Ms of the light receiving surface is schematically illustrated.

The solid state image sensor 44 has a light receiving unit 44a that receives light and performs the photoelectric conversion on the light to generate an electric signal. A sensor side land 44b is provided at the light receiving surface provided with the light receiving unit 44a. An inner lead 54a of the flexible substrate 45 is electrically and mechanically connected to the sensor side land 44b. The glass rid 49 is bonded to the solid state image sensor 44, with the light receiving unit 44a (center Ms) of the solid state image sensor 44 covered therewith.

The flexible substrate 45 is a flexible printed circuit board extending to a surface side opposite to the light receiving surface of the solid state image sensor 44 provided with the light receiving unit 44a. The inner lead 54a is bent approximately 90 degrees at a distal end of the flexible substrate 45, and fixed to the solid state image sensor 44 and the flexible substrate 45 by a sealing resin 54c. A back surface of the solid state image sensor 44 and a side surface of a distal end side of the multi-layer substrate 46 are bonded together by the adhesive 54b. Peripheries of the sensor side land 44b of the solid state image sensor 44 and the distal end side of the flexible substrate 45 are sealed with the sealing resin 54c. A surface f2 of the flexible substrate 45 that is in contact with the solid state image sensor 44 is coupled via an adhesive 54d so as to face a connection surface S2 of the multi-layer substrate 46. As illustrated in FIG. 7, a wiring pattern 45a is formed on a surface f1 of the flexible substrate 45 opposite to the surface f2 that is in contact with the solid state image sensor 44, and the wiring pattern 45a is protected by a resist layer 61.

The multi-layer substrate 46 is formed in such a manner that a plurality of substrates with circuit patterns is stacked. A recessed portion 60 is formed on a proximal end side relative to a connection portion between the flexible substrate 45 and the multi-layer substrate 46 on the connection surface S2 to which the flexible substrate 45 is connected. The five electronic components 55 to 59 are mounted on the multi-layer substrate 46. The electronic components 58 and 59 are mounted in the recessed portion 60, and the electronic components 55 to 57 are mounted on a surface S1 opposite to the connection surface S2. As used herein, the electronic component 56 is an active component, and the electronic components 55 and 57 to 59 are passive components. The electronic component 58 mounted in the recessed portion 60 is connected to connection lands 74a and 74b, and the electronic component 59 is connected to connection lands 73a and 73b. The electronic component 55 mounted on the surface S1 is connected to connection lands 70a and 70b, the electronic component 56 is connected to connection lands 75a, 75b, 75c, 75d, 75e, and 75f, and the electronic component 57 is connected to connection lands 71a and 71b.

The depth h1 of the recessed portion 60 from the connection surface S2 is less than the height h2 of the electronic component 58 that is the taller of the electronic components 58 and 59 mounted in the recessed portion 60. The electronic component 58 has such a height that the electronic component 58 does not project from the surface f1 of the flexible substrate 45 opposite to the surface f2 to which the multi-layer substrate 46 is connected. In other words, the height h2 of the electronic component 58 is equal to or less than the height h3 from the bottom of the recessed portion 60 to the surface f1 of the flexible substrate 45. The depth h1 of the recessed portion 60 and the height h2 of the electronic component 58 are set in the above-mentioned range, whereby breakage of the multi-layer substrate 46 can be prevented while an increase in the diameter of the imaging unit 40 is suppressed.

Cable connection lands 72b, 72c-1, and 72c-2 are provided on a proximal end side of the surface S1 of the multi-layer substrate 46. Cable connection lands 72a and 72c-3 are provided on a proximal end side relative to the recessed portion 60 on the connection surface S2 of the multi-layer substrate 46. An image signal cable 48a is connected to the cable connection land 72a, a drive signal cable (not illustrated) is connected to the cable connection land 72b, and a power cable 48c is connected to the cable connection lands 72c-1 to 72c-3. The drive signal cable through which a drive signal that drives the solid state image sensor 44 is transmitted and the image signal cable 48a that transmits the image signal are respectively connected to the different surfaces S1 and S2 of the multi-layer substrate 46, whereby interference of the drive signal in the image signal can be suppressed, and noise can be reduced.

Preferably, the proximal end side of the multi-layer substrate 46 provided with the cable connection lands 72b, 72c-1, and 72c-2 is a layer different from the layer provided with connection lands 70, 71, and 75 for the electronic components 55 to 57. As illustrated in FIG. 4, the proximal end side of the multi-layer substrate 46 provided with the cable connection lands 72b, 72c-1, and 72c-2 is higher than the layer provided with the connection lands 70, 71, and 75 for the electronic components 55 to 57 by h5. In other words, the proximal end side of the multi-layer substrate 46 is thicker than the part on which the electronic components 55 to 57 are mounted since many substrates are stacked. Owing to the thickness of the proximal end side of the multi-layer substrate 46, a possible short circuit between cable connection lands 72 and the connection lands 71 for the electronic component 57 due to solder spills can be prevented when the signal cables 48 are connected to the cable connection lands 72 by solder. Similarly, the proximal end side of the multi-layer substrate 46 provided with the cable connection lands 72a and 72c-3 is preferably higher than the layer provided with connection lands 73 and 74 for the electronic components 58 and 59 by h4. In other words, the proximal end side of the multi-layer substrate 46 is preferably thicker than the part on which the electronic components 58 and 59 are mounted since many substrates are stacked.

Next, a method of manufacturing the imaging unit 40 according to the present embodiment will be described. FIG. 8 is a flowchart describing steps of manufacturing the imaging unit 40 according to the embodiment of the present invention. In the present embodiment, a plurality of multi-layer substrates 46 is simultaneously produced on a single aggregate substrate, whereby the multi-layer substrate 46 that constitutes the imaging unit 40 is manufactured.

The plurality of electronic components 55, 56, and 57 and the signal cables 48 are respectively mounted on the plurality of connection lands 70, 71, and 75 and the cable connection lands 72 provided on a principal surface (surface that serves as the surface S1 of the multi-layer substrate 46) of the aggregate substrate, and the plurality of electronic components 58 and 59 and the signal cables 48 are respectively mounted on the plurality of connection lands 73 and 74 and the cable connection lands 72 provided on a back surface (surface that serves as the connection surface S2 of the multi-layer substrate 46) (step S1).

After the electronic components are mounted on the aggregate substrate, a dicing tape is attached to the back surface (surface that serves as the connection surface S2 of the multi-layer substrate 46), and the aggregate substrate is fixed (step S2). On the back surface of the aggregate substrate, the electronic components 58 and 59 are mounted in the recessed portion 60, and the taller electronic component 58 slightly projects from a horizontal plane of the back surface of the aggregate substrate. The dicing tape is attached so as to come into contact with the back surface of the aggregate substrate and the electronic components 58 and 59. In order to ensure that the dicing tape is firmly attached to the electronic component 58 for enabling stable dicing, preferably, the height of the electronic component 58 projecting from the back surface of the aggregate substrate is substantially equal to the thickness of the dicing tape. In the present embodiment, the electronic components 58 and 59 having different heights are mounted in the recessed portion 60. Preferably, however, electronic components having the same height are mounted in the recessed portion 60. When the electronic components having the same height are mounted, the area fixed by the dicing tape is increased, and the stable dicing for singulation is enabled.

After the aggregate substrate is fixed by the dicing tape, the aggregate substrate is cut and singulated into the multi-layer substrates 46 (step S3). After the singulation, the dicing tape attached to the multi-layer substrate 46 is peeled off (step S4). In order to easily peel off the dicing tape, UV radiation or heat treatment is preferably performed in accordance with the type of the used dicing tape before the dicing tape is peeled off.

After the dicing tape is peeled off, the multi-layer substrate 46 is connected to the flexible substrate 45 and the solid state image sensor 44 (step S5). The multi-layer substrate 46 is arranged so that the connection surface S2 faces the surface f2 of the flexible substrate 45, and electrically and mechanically connected to the flexible substrate 45. The multi-layer substrate 46 and the flexible substrate 45 are preferably connected in such a manner that a connection land (not illustrated) provided on the connection surface S2 of the multi-layer substrate 46 and a connection land (not illustrated) provided on the flexible substrate 45 are coupled by a bump such as an Au bump and a solder bump, and bonded and fixed together by the adhesive 54d. The side surface of the distal end side of the multi-layer substrate 46 is bonded to the surface of the solid state image sensor 44 opposite to the light receiving surface by the adhesive 54b.

In the above-mentioned manner, the imaging unit 40 according to the present embodiment is produced. In the first embodiment, the recessed portion 60 in which the electronic components are mounted is provided on the connection surface of the multi-layer substrate 46 connected to the flexible substrate 45. The depth of the recessed portion 60 from the connection surface S2 is less than the height of the electronic component that is the taller of the electronic components mounted in the recessed portion 60, and each of the mounted electronic components has such a height that the electronic component does not project from the surface f1 of the flexible substrate 45 opposite to the surface f2 connected to the multi-layer substrate 46. Consequently, breakage of the multi-layer substrate 46 can be prevented while an increase in the diameter of the imaging unit 40 is suppressed.

### (First Variation)

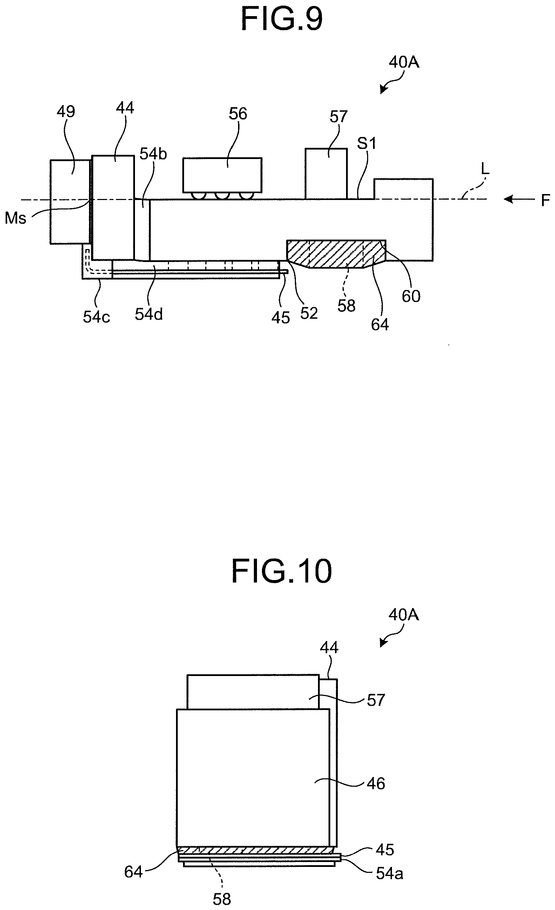

During the production of the imaging unit 40, after the electronic components are mounted on the aggregate substrate (step S1), the recessed portion 60 in which the electronic components 58 and 59 have been mounted may be filled and sealed with a sealing resin. FIG. 9 is a left side view of an imaging unit according to a first variation of the embodiment of the present invention. FIG. 10 is another view of FIG. 9 seen in the direction of arrow F.

In an imaging unit 40A according to the first variation, the recessed portion 60 is filled with a sealing resin 64. As illustrated in FIGS. 9 and 10, the sealing resin 64 is applied in the recessed portion 60 so as to cover a side surface of the electronic component 58. After the electronic components are mounted on the aggregate substrate, the recessed portion 60 in which the electronic components 58 and 59 have been mounted is filled with the sealing resin 64. After that, the dicing tape is attached to the back surface of the aggregate substrate, and the aggregate substrate is diced. Since the recessed portion 60 is filled with the sealing resin 64 after the electronic components 58 and 59 are mounted in the recessed portion, connection reliability of the electronic components 58 and 59 is maintained. In addition, the area fixed by the dicing tape is increased since a space between the electronic components 58, 59, and the recessed portion 60 is sealed with the sealing resin, whereby the stable dicing is enabled. The procedure subsequent to the dicing is just the same as the above-mentioned one; that is, the dicing tape is peeled off, and the multi-layer substrate 46 is connected to the flexible substrate 45 or the like for producing the imaging unit 40A.

### (Second Variation)

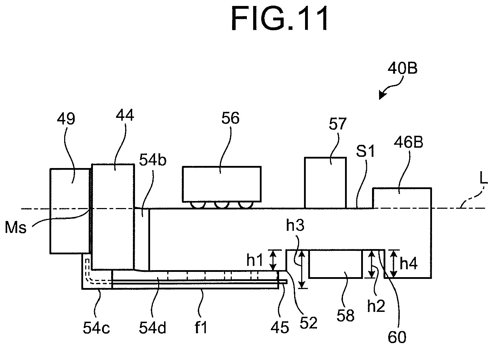

In the embodiment, the connection surface S2 of the multi-layer substrate 46 is configured such that the part facing the flexible substrate 45 and the part provided with the cable connection lands 72 are at the same height. Alternatively, the height of the proximal end side of the multi-layer substrate 46 provided with the cable connection lands 72 may differ from the height of the part facing the flexible substrate. FIG. 11 is a left side view of an imaging unit according to a second variation of the embodiment of the present invention.

In an imaging unit 40B according to the second variation, as illustrated in FIG. 11, the height h4 from the bottom of the recessed portion 60 on the proximal end side of a multi-layer substrate 46B is higher than the height h1 from the bottom of the recessed portion 60 on the part facing the flexible substrate 45. Substrates are further stacked on the proximal end side, whereby the thickness of the proximal end side of the multi-layer substrate 46B can be increased. The height h4 from the bottom of the recessed portion 60 on the proximal end side of the multi-layer substrate 46B is equal to or less than the height h2 of the electronic component 58, that is, equal to or less than the height h3 from the bottom of the recessed portion 60 to the surface f1 of the flexible substrate 45. In consideration of the dicing for the singulation to obtain the multi-layer substrate 46B, the height h4 from the bottom of the recessed portion 60 on the proximal end side of the multi-layer substrate 46B is preferably equal to the height h2 of the electronic component 58. When the height h4 from the bottom of the recessed portion 60 on the proximal end side of the multi-layer substrate 46B is equal to the height h2 of the electronic component 58, the area fixed by the dicing tape is increased, and the stable dicing is enabled.

### (Third Variation)

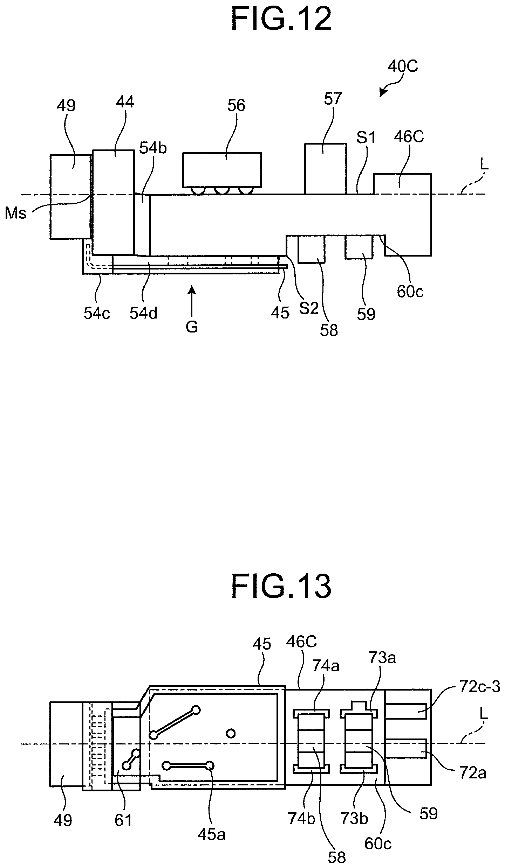

In the present embodiment, the electronic components 58 and 59 having different heights are mounted in the recessed portion 60 side by side in a lateral direction. Alternatively, the electronic components 58 and 59 may be mounted side by side in a longitudinal direction of the multi-layer substrate. FIG. 12 is a left side view of an imaging unit according to a third variation of the embodiment of the present invention. FIG. 13 is another view of FIG. 12 seen in the direction of arrow G.

In an imaging unit 40C according to the third variation, as illustrated in FIGS. 12 and 13, the electronic components 58 and 59 are mounted in a recessed portion 60c of a multi-layer substrate 46C side by side in the longitudinal direction. Since the dicing tape is attached in the longitudinal direction of the multi-layer substrate 46C, the electronic components 58 and 59 having different heights and mounted in the longitudinal direction allow the dicing tape to fix a large area when the dicing tape is attached to the electronic components 58 and 59, whereby the stable dicing is enabled. In a case where the connection surface S2 of the multi-layer substrate is configured such that the part provided with the cable connection lands 72 is higher than the part facing the flexible substrate 45 as in the second variation, the taller electronic component 58 is preferably mounted on the proximal end side of the multi-layer substrate.

### (Fourth Variation)

In the present embodiment, the connection lands connected to the electronic component serving as the passive component are arranged so that the two connection lands face each other in a long-side direction of the electronic component, the land length of the connection land is longer than the short-side length of the mounted electronic component, and the arrangement distance between the two connection lands is longer than the long-side length of the electronic component. In order to enable the electronic components 58 and 59 to be easily mounted in the recessed portion 60, and in order to reduce the diameter of the imaging unit and reduce the size of the imaging unit (shorten the length in an axial direction), the two connection lands may be arranged so that the arrangement distance between the two connection lands is equal to the long-side length of the mounted electronic component.

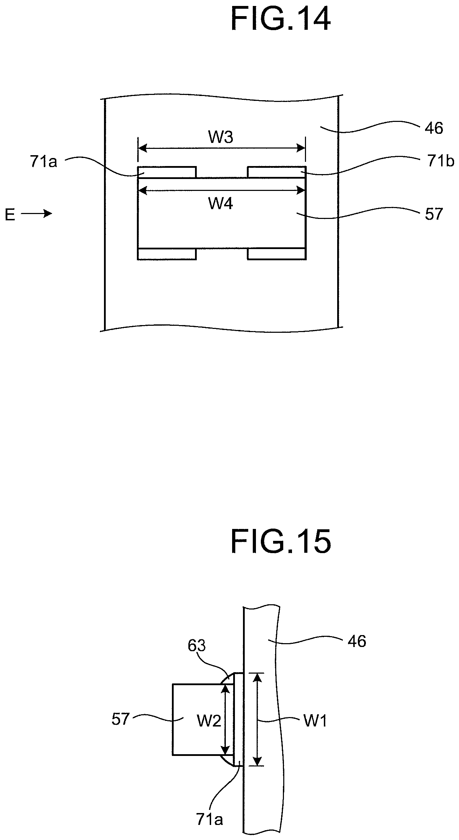

FIG. 14 is a partial top view of an adjacent region of the electronic component 57 of the imaging unit according to a fourth variation of the embodiment of the present invention. FIG. 15 is another view of FIG. 14 seen in the direction of arrow E. Solder 63 is not illustrated in FIG. 14 for the convenience of describing the sizes of the electronic component 57 and the connection lands 71. In the fourth variation, the two connection lands 71a and 71b are arranged so that the land length W1 of each of the two connection lands 71a and 71b is longer than the short-side length W2 of the electronic component 57, and the arrangement distance W3 between the two connection lands 71a and 71b is equal to the long-side length W4 of the electronic component 57. In the present embodiment, as illustrated in FIG. 3, the connection lands 71a and 71b arranged so as to protrude from the electronic component 57 in the long-side direction are coupled to the electronic component 57 by the solder. Since the long side of the electronic component 57 is the longest of the long sides of the electronic components mounted on the surface S1 of the multi-layer substrate 46, the imaging unit can be reduced in diameter when the sizes of and arrangement distance between the connection lands 71a and 71b for mounting the electronic component 57 are set as mentioned above. Similarly, the electronic components 58 and 59 mounted in the recessed portion 60 are arranged and connected so that the land length of each of the connection lands 73a and 73b and the land length of each of the connection lands 74a and 74b are respectively longer than the short-side lengths of the electronic components 58 and 59, and the arrangement distance between the connection lands 73a and 73b and the arrangement distance between the connection lands 74a and 74b are respectively equal to the long-side lengths of the electronic components 58 and 59. Consequently, the electronic components can be easily mounted in the recessed portion 60, and the imaging unit can be reduced in size (length in the axial direction is shortened).

### Reference Signs List

- 1: ENDOSCOPE DEVICE

- 2: ENDOSCOPE
- 3a: CURVE PORTION
- 3b: DISTAL END PORTION
- 3c: FLEXIBLE PIPE PORTION
- 4: OPERATING UNIT
- 4a: TREATMENT TOOL INSERTION OPENING
- 5: UNIVERSAL CODE
- 6: CONNECTOR
- 7: LIGHT SOURCE DEVICE
- 8: PROCESSOR
- 10: DISPLAY DEVICE
- 40,: 40A, 40B, 40C IMAGING UNIT
- 41: DISTAL END PORTION BODY
- 41a: ADHESIVE
- 42: COVERING PIPE
- 43: LENS UNIT
- 43a-1 to 43a-4: OBJECTIVE LENS
- 43b: LENS HOLDER
- 44: SOLID STATE IMAGE SENSOR
- 44a: LIGHT RECEIVING UNIT
- 44b: SENSOR SIDE LAND
- 45: FLEXIBLE SUBSTRATE
- 45a: WIRING PATTERN
- 46, 46B, 46C: MULTI-LAYER SUBSTRATE
- 47: ELECTRIC CABLE BUNDLE
- 48: SIGNAL CABLE
- 48a: IMAGE SIGNAL CABLE
- 48c: POWER CABLE
- 49: GLASS RID
- 50: HEAT SHRINKABLE TUBE
- 51: ADHESIVE RESIN
- 52: REINFORCING MEMBER
- 53: SOLID STATE IMAGE SENSOR HOLDER
- 54a: INNER LEAD
- 54b, 54d: ADHESIVE
- 54c: SEALING RESIN
- 55 to 59: ELECTRONIC COMPONENT
- 70a, 70b, 71a, 71b, 73a, 73b, 74a, 74b, 75a, 75b, 75c, 75d, 75e, 75f: CONNECTION LAND
- 72a, 72b, 72c-1, 72c-2, 72c-3: CABLE CONNECTION LAND
- 60: RECESSED PORTION
- 61: RESIST LAYER
- 63: SOLDER
- 81: CURVE PIPE
- 82: CURVE WIRE

## Claims

1. An imaging unit (40) comprising:
a solid state image sensor (44) configured to receive light and perform photoelectric conversion on the light to generate an electric signal;
a flexible substrate (45) including one end connected to a light receiving surface of the solid state image sensor (44), the flexible substrate (45) extending to a surface side opposite to the light receiving surface;
a multi-layer substrate (46) connected to a surface of the flexible substrate (45), the surface of the flexible substrate (45) being a surface to which the solid state image sensor (44) is connected, the multi-layer substrate (46) including connection lands on which a plurality of electronic components (55 to 59) are mounted; and
a bump by which connection lands provided on the surface of the flexible substrate (45) and connection lands provided on a surface of the multi-layer substrate (46) facing the surface of the flexible substrate (45) are coupled so that the multi-layer substrate (46) is electrically and mechanically connected to the flexible substrate (45), wherein
the multi-layer substrate (46) includes, on a proximal end side relative to a connection portion between the flexible substrate (45) and the multi-layer substrate (46) on a connection surface to which the flexible substrate (45) is connected, a recessed portion (60) in which at least one of the electronic components (55 to 59) is mounted,
a depth of the recessed portion (60) from the connection surface is less than a height of the at least one of the electronic components (55 to 59) mounted in the recessed portion (60),
each electronic component (55 to 59) mounted in the recessed portion (60) on the connection surface of the multi-layer substrate (46) connected to the flexible substrate (45) is configured not to project from a surface of the flexible substrate (45) opposite to the connection surface,
cable connection lands (72) are formed on proximal end sides relative to the recessed portion (60) on the connection surface of the multi-layer substrate (46) and a surface of the multi-layer substrate (46) opposite to the connection surface, and
a drive signal cable and an image signal cable (48) are connected to the cable connection lands (72) formed on the different surfaces of the multi-layer substrate (46), respectively.

2. The imaging unit (40) according to claim 1, wherein
two or more of the electronic components (55 to 59) having different heights are mounted in the recessed portion (60), and the tallest electronic component is configured not to project from the surface of the flexible substrate (45) opposite to the connection surface.

3. The imaging unit (40) according to claim 2, wherein
the two electronic components (55 to 59) having different heights are mounted in the recessed portion (60), and the two electronic components (55 to 59) are mounted side by side in a longitudinal direction of the multi-layer substrate (46).

4. An endoscope (2) comprising:
an insertion portion (3) including a distal end provided with the imaging unit (40) according to claim 1.

5. A method of manufacturing the imaging unit (49) according to claim 1, the method comprising:
a mounting step of mounting a plurality of electronic components (55 to 59) in a recessed portion (60) of an aggregate substrate;
a sealing step of filling and sealing, with a sealing resin (54c), the recessed portion (69) in which the electronic components (55 to 59) have been mounted;
a cutting step of fixing, with a dicing tape, a surface of the aggregate substrate provided with the recessed portion (60), and cutting the fixed aggregate substrate at a predetermined position to perform singulation to obtain a multi-layer substrate (46);
a peeling step of peeling off the dicing tape from the multi-layer substrate (46) obtained by the singulation; and
a connecting step of connecting the multi-layer substrate (46) to a surface of a flexible substrate (45), the surface of the flexible substrate (45) being a surface to which a solid state image sensor (44) is connected, the flexible substrate (45) including one end connected to a light receiving surface of the solid state image sensor (44), the flexible substrate (45) extending to a surface side opposite to the light receiving surface.

## Patentansprüche

1. Abbildungseinheit (40), die umfasst:
einen Festkörper-Bildsensor (44), der so konfiguriert ist, dass er Licht empfängt und an dem Licht eine photoelektrische Umwandlung durchführt, um ein elektrisches Signal zu erzeugen;
ein flexibles Substrat (45) mit einem Ende, das mit einer Lichtempfangsfläche des Festkörper-Bildsensors (44) verbunden ist, wobei sich das flexible Substrat (45) zu einer der Lichtempfangsfläche gegenüberliegenden Oberflächenseite erstreckt;
ein Mehrschichtsubstrat (46), das mit einer Oberfläche des flexiblen Substrats (45) verbunden ist, wobei die Oberfläche des flexiblen Substrats (45) eine Oberfläche ist, mit der der Festkörper-Bildsensor (44) verbunden ist, wobei das Mehrschichtsubstrat (46) Verbindungsanschlüsse umfasst, auf denen eine Vielzahl von elektronischen Komponenten (55 bis 59) angebracht ist; und
einen Höcker, durch den Verbindungsanschlüsse, die auf der Oberfläche des flexiblen Substrats (45) vorgesehen sind, und Verbindungsanschlüsse, die auf einer Oberfläche des Mehrschichtsubstrats (46) vorgesehen sind, die der Oberfläche des flexiblen Substrats (45) zugewandt ist, gekoppelt sind, so dass das Mehrschichtsubstrat (46) elektrisch und mechanisch mit dem flexiblen Substrat (45) verbunden ist, wobei
das Mehrschichtsubstrat (46) an einer proximalen Endseite relativ zu einem Verbindungsabschnitt zwischen dem flexiblen Substrat (45) und dem Mehrschichtsubstrat (46) auf einer Verbindungsfläche, mit der das flexible Substrat (45) verbunden ist, einen vertieften Abschnitt (60) aufweist, in dem mindestens eines der elektronischen Bauteile (55 bis 59) angebracht ist,
eine Tiefe des vertieften Abschnitts (60) von der Anschlussfläche kleiner ist als eine Höhe der mindestens einen der elektronischen Komponenten (55 bis 59), die in dem vertieften Abschnitt (60) angebracht sind,
jedes elektronische Bauteil (55 bis 59), das in dem vertieften Abschnitt (60) auf der Verbindungsfläche des mehrschichtigen Substrats (46), das mit dem flexiblen Substrat (45) verbunden ist, angebracht ist, so konfiguriert ist, dass es nicht von einer Oberfläche des flexiblen Substrats (45) gegenüber der Verbindungsfläche vorsteht,
Kabelanschlussflächen (72) an proximalen Endseiten relativ zu dem vertieften Abschnitt (60) auf der Verbindungsfläche des mehrschichtigen Substrats (46) und einer der Verbindungsfläche gegenüberliegenden Fläche des mehrschichtigen Substrats (46) ausgebildet sind, und
ein Steuersignalkabel und ein Bildsignalkabel (48) mit den Kabelanschlussflächen (72) verbunden sind, die jeweils auf den verschiedenen Oberflächen des Mehrschichtsubstrats (46) ausgebildet sind.

2. Abbildungseinheit (40) nach Anspruch 1, wobei
zwei oder mehr der elektronischen Komponenten (55 bis 59) mit unterschiedlichen Höhen in dem vertieften Abschnitt (60) angebracht sind und die höchste elektronische Komponente so konfiguriert ist, dass sie nicht von der Oberfläche des flexiblen Substrats (45) gegenüber der Verbindungsfläche vorsteht.

3. Abbildungseinheit (40) nach Anspruch 2, wobei
die zwei elektronischen Komponenten (55 bis 59), die unterschiedliche Höhen haben, in dem vertieften Abschnitt (60) angebracht sind, und die zwei elektronischen Komponenten (55 bis 59) nebeneinander in einer Längsrichtung des mehrschichtigen Substrats (46) angebracht sind.

4. Endoskop (2), umfassend:
ein Einführteil (3) mit einem distalen Ende, das mit der Abbildungseinheit (40) nach Anspruch 1 versehen ist.

5. Verfahren zur Herstellung der Abbildungseinheit (49) nach Anspruch 1, wobei das Verfahren umfasst:
einen Montageschritt eines Montierens einer Vielzahl von elektronischen Bauteilen (55 bis 59) in einem vertieften Abschnitt (60) eines Aggregatsubstrats;
einen Versiegelungsschritt eines Füllens und Versiegelns mit einem Versiegelungsharz (54c) des vertieften Abschnitts (69), in dem die elektronischen Komponenten (55 bis 59) montiert wurden;
einen Schneideschritt eines Fixierens einer Oberfläche des Aggregatsubstrats, das mit dem vertieften Abschnitt (60) versehen ist, mit einem Klebeband, und eines Schneidens des fixierten Aggregatsubstrats an einer vorbestimmten Position, um eine Vereinzelung durchzuführen, um ein mehrlagiges Substrat (46) zu erhalten;
einen Abziehschritt eines Abziehens des Klebebands von dem durch die Vereinzelung erhaltenen mehrschichtigen Substrat (46); und
einen Verbindungsschritt eines Verbindens des Mehrschichtsubstrats (46) mit einer Oberfläche eines flexiblen Substrats (45), wobei die Oberfläche des flexiblen Substrats (45) eine Oberfläche ist, mit der ein Festkörper-Bildsensor (44) verbunden ist, wobei das flexible Substrat (45) ein Ende aufweist, das mit einer Lichtempfangsfläche des Festkörper-Bildsensors (44) verbunden ist, wobei sich das flexible Substrat (45) zu einer Oberflächenseite gegenüber der Lichtempfangsfläche erstreckt.

## Revendications

1. Unité d'imagerie (40) comprenant :
un capteur d'image à semi-conducteur (44) conçu pour recevoir la lumière et procéder à la conversion photoélectrique sur la lumière pour produire un signal électrique ;
un substrat souple (45) comprenant une extrémité connectée à une surface de réception de lumière du capteur d'image à semi-conducteur (44), le substrat souple (45) s'étendant sur un côté de surface en regard de la surface de réception de lumière ;
un substrat multicouche (46) connecté à une surface du substrat souple (45), la surface du substrat souple (45) étant une surface à laquelle est connecté le capteur d'image à semi-conducteur (44), le substrat multicouche (46) comprenant des zones de connexion sur lesquelles est montée une pluralité de composants électriques (55 à 59) ; et
une bosse par laquelle les zones de connexion disposées sur la surface du substrat souple (45) et les zones de connexion disposées sur une surface du substrat multicouche (46) en regard du substrat souple (45) sont reliées de manière à ce que le substrat multicouche (46) soit connecté électriquement et mécaniquement au substrat souple (45),
le substrat multicouche (46) comprenant, sur un côté d'extrémité proximale par rapport à une partie de connexion entre le substrat souple (45) et le substrat multicouche (46) sur une surface de connexion à laquelle est connecté le substrat souple (45), une partie en retrait (60) dans laquelle est monté au moins l'un des composants électroniques (55 à 59),
une profondeur de la partie en retrait (60) depuis la surface de connexion étant inférieure à une hauteur d'au moins l'un des composants électroniques (55 à 59) montés dans la partie en retrait (60),
chaque composant électronique (55 à 59) monté dans la partie en retrait (60) sur la surface de connexion du substrat multicouche (46) connecté au substrat souple (45) étant conçu pour ne pas faire saillie d'une surface du substrat souple (45) en regard de la surface de connexion,
les zones de connexion de câble (72) étant formées sur des côtés d'extrémité proximale par rapport à la partie en retrait (60) sur la surface de connexion du substrat multicouche (46) et une surface du substrat multicouche (46) en regard de la surface de connexion, et
un câble de signal d'attaque et un câble de signal d'image (48) étant connectés aux zones de connexion de câble (72) formées sur les différentes surfaces du substrat multicouche (46), respectivement.

2. Unité d'imagerie (40) selon la revendication 1,
au moins deux des composants électroniques (55 à 59) ayant différentes hauteurs sont montés dans la partie en retrait (60), le composant électronique le plus haut étant conçu pour ne pas faire saillie de la surface du substrat souple (45) en regard de la surface de connexion.

3. Unité d'imagerie (40) selon la revendication 2,
les deux composants électroniques (55 à 59) ayant des hauteurs différentes sont montés dans la partie en retrait (60), et les deux composants électroniques (55 à 59) sont montés côte à côté dans le sens longitudinal du substrat multicouche (46).

4. Endoscope (2) comprenant :
une partie d'insertion (3) comprenant une extrémité distale dotée de l'unité d'imagerie (40) selon la revendication 1.

5. Procédé de fabrication de l'unité d'imagerie (49) selon la revendication 1, le procédé comprenant :
une étape de montage d'une pluralité de composants électroniques (55 à 59) dans une partie en retrait (60) dans un substrat agrégé ;
une étape d'étanchéité de remplissage et d'étanchéité, au moyen d'une résine hermétique (54c), la partie en retrait (69) dans laquelle ont été montés les composants électroniques (55 à 59) ;
une étape de découpe pour fixer, à l'aide d'un ruban de découpage, une surface du substrat agrégé doté de la partie en retrait (60), et pour couper le substrat agrégé fixé dans une position prédéfinie pour procéder à la séparation afin d'obtenir un substrat multicouche (46) ;
une étape de pelage pour peler le ruban de découpage du substrat multicouche (46) obtenu par la séparation ; et
une étape de connexion pour connecter le substrat multicouche (46) à une surface d'un substrat souple (45), la surface du substrat souple (45) étant une surface à laquelle est connecté un capteur d'image à semi-conducteur (44), le substrat souple (45) comprenant une extrémité connectée à une surface de réception de lumière du capteur d'image à semi-conducteur (44), le substrat souple (45) s'étendant sur un côté de surface en regard de la surface de réception de lumière.
